Europäisches Patentamt

(19) European Patent Office

Office européen des brevets

(11) Publication number: **0 005 287**
**B1**

# (12) EUROPEAN PATENT SPECIFICATION

(45) Date of publication of patent specification: **09.09.81**

(21) Application number: **79200176.0**

(22) Date of filing: **10.04.79**

(51) Int. Cl.³: **C 07 C 15/00, C 07 C 1/20,
B 01 J 29/28 //C07C43/04,
C07C41/01**

(54) A process for the preparation of an aromatic hydrocarbon mixture.

| | |
|---|---|
| (30) Priority: **21.04.78 NL 7804269** | (73) Proprietor: **SHELL INTERNATIONALE RESEARCH MAATSCHAPPIJ B.V.**<br>**Carel van Bylandtlaan 30**<br>**NL-2596 HR DEN HAAG (NL)** |
| (43) Date of publication of application:<br>**14.11.79 Bulletin 79/23** | |
| (45) Publication of the grant of the European patent:<br>**09.09.81 Bulletin 81/36** | (72) Inventor: **Schaper, Lambert**<br>**Badhuisweg 3**<br>**NL-1031 CM Amsterdam (NL)**<br>Inventor: **Sie, Swan Tiong**<br>**Badhuisweg 3**<br>**NL-1031 CM Amsterdam (NL)** |
| (84) Designated Contracting States:<br>**BE DE FR GB IT** | |
| (56) References cited:<br>**DE - A - 2 755 770**<br>**GB - A - 403 402**<br>**GB - A - 1 446 522**<br>**US - A - 3 998 899** | (74) Representative: **Keuzenkamp, Abraham et al,**<br>**c/o Shell Internationale Research Maatschappij**<br>**B.V. P.O. Box 302**<br>**NL-2501 CH 's-Gravenhage (NL)** |

Courier Press, Leamington Spa, England.

# 0 005 287

## A process for the preparation of an aromatic hydrocarbon mixture

The invention relates to a process for the preparation of an aromatic hydrocarbon mixture from methanol.

Aromatic hydrocarbon mixtures are widely used as gasoline. As a rule they are obtained by catalytic reforming of petroleum-based aliphatic hydrocarbon mixtures which boil in the gasoline range.

In US—A— 3,998,899 a two-stage process for converting $C_1$ to $C_3$ alcohols to gasoline boiling range products is described. Initially ethers are formed in a first catalyst stage followed by conversion of the ether product in a second stage using a crystalline aluminosilicate zeolite catalyst of the ZSM—5 type.

An investigation by the Applicant into the preparation of aromatic hydrocarbon mixtures from aliphatic compounds has shown that certain crystalline silicates recently synthesized for the first time by the Applicant are suitable for use as catalysts for the preparation of aromatic hydrocarbon mixtures from methanol. By contacting methanol at elevated temperature with a catalyst containing one of these crystalline silicates, an aromatic hydrocarbon mixture boiling in the gasoline range is obtained in a high yield. The novel crystalline silicates which have the property of catalysing the above-mentioned conversion are characterized as follows:

a) they are thermally stable up to temperatures above 600°C,

b) they are capable, after dehydration at 400°C in vacuo, of absorbing more than 3% by weight of water at 25°C and saturated water vapour pressure, and

c) they possess in the dehydrated form the following overall composition, expressed in moles of the oxides, $(1.0 \pm 0.3)(R)_{2/n}O.$ [a $Fe_2O_3$. b $Al_2O_3$. c $Ga_2O_3$].

y (d $SiO_2$. e $GeO_2$), wherein

R = one or more mono- or bivalent cations,

$a \geqslant 0.1$,

$b \geqslant 0$,

$c \geqslant 0$,

$a + b + c = 1$,

$y \geqslant 10$,

$d \geqslant 0.1$,

$e \geqslant 0$,

$d + e = 1$, and

n = the valency of R.

A drawback of the above-mentioned single-stage process for the preparation of an aromatic hydrocarbon mixture from methanol is that a large amount of heat is liberated in the reaction. As a result it is extremely difficult to obviate undesirable temperature rises which adversely affect the catalyst life and the product composition.

Prolonged investigation in respect of this subject by the Applicant has now shown that this drawback can be obviated by carrying out the process in two stages, methanol being converted into dimethyl ether in the first stage and dimethyl ether being converted into an aromatic hydrocarbon mixture in the second stage. The first stage of the process should be carried out by contacting methanol at elevated temperature with a dehydration catalyst, as is for instance described in GB—A— 403 402 and the second stage by contacting dimethyl ether at elevated temperature with a catalyst which contains a crystalline silicate as defined above.

The present patent application therefore relates to a process for the preparation of an aromatic hydrocarbon mixture from methanol, which process is carried out in two stages in which dimethyl ether is prepared in the first stage by contacting methanol at elevated temperature with a dehydration catalyst and in which in the second stage an aromatic hydrocarbon mixture is prepared by contacting dimethyl ether form the first stage at elevated temperature with a catalyst which contains a crystalline silicate as defined above.

Methanol used as starting material in the process according to the invention may be very suitably prepared by contacting a mixture of carbon monoxide and hydrogen (called synthesis gas) at elevated temperature with a methanol-synthesis catalyst. The methanol preparation is preferably effected by contacting the synthesis gas at a temperature between 200 and 400°C and a pressure between 25 and 425 bar with a catalyst containing zinc and/or copper. The preparation of the synthesis gas required for the methanol synthesis is preferably effected by gasification of coal or liquid petroleum fractions, or starting from natural gas, by catalytic steam-reforming or partial oxidation.

In the first stage of the process according to the invention dimethyl ether should be prepared by contacting methanol at elevated temperature with a dehydration catalyst. Suitable dehydration catalysts are liquid acids such as sulphuric acid and phosphoric acid and solid inorganic and organic catalysts, such as phosphoric acid supported on kieselguhr, silica-aluminas having a large surface, acidic aluminas, acid-treated clays, bauxite and polystyrene sulphonic acids. The preferred catalyst is gamma alumina. The first stage of the process is preferably carried out at a temperature between 250 and 400°C, a pressure between 1 and 50 bar and a space velocity between 0.63 and 2531.1$^{-1}$ h$^{-1}$. In

2

the first stage of the process a reaction product is obtained which consists of a mixture of dimethyl ether, water and unconverted methanol. If desired, this mixture can be used as such as feed for the second stage. Preferably, however, at least part of the water and in particular essentially all the water is removed therefrom before the mixture is used as feed for the second stage. Further it is preferred to separate also non-converted methanol from the reaction product of the first stage and to recycle at least part thereof.

In the second stage of the process according to the invention an aromatic hydrocarbon mixture is prepared from dimethyl ether originating from the first stage while using a catalyst which contains a crystalline silicate belonging to a special class. These silicates effect a high conversion of aliphatic hydrocarbons into aromatic hydrocarbons in commercially desired yields and are generally highly active in conversion reactions involving aromatic hydrocarbons. In the process according to the invention it is preferred to use silicates which do not contain gallium and germanium, in other words, silicates of which c and e in the above overall composition equal 0. Silicates of this type are the subject of the Netherlands patent application No. 7613957. It is also preferred to use silicates of which a in the above-mentioned overall composition is greater than 0.5. Silicates which do not contain aluminium, in other words, silicates of which b in the above-mentioned overall composition is 0 are particularly preferred. As regards y it may be noted that in the silicates used in the process according to the invention, this term should preferably be less than 800 and in particular less than 600. Finally, in the process according to the invention it is preferred to use silicates of which the X-ray powder diffraction pattern contains inter alia the reflections mentioned in Table A of the Netherlands patent application No. 7613957. The second stage of the process according to the invention is preferably carried out at a temperature between 250 and 500°C and in particular between 325 and 450°C, a pressure between 0.5 and 100 bar and in particular between 1 and 40 bar, and a space velocity between 0.1 and 20 $1.1^{-1}.h^{-1}$ and in particular between 0.5 and 10 $1.1^{-1}.h^{-1}$.

By making use of the two-stage embodiment as proposed in this application in the preparation of an aromatic hydrocarbon mixture starting from methanol, the temperature control of the process is considerably simplified. A further simplification in this respect can be achieved by adding a gaseous light hydrocarbon fraction as diluent to the feed for the second stage. The process according to the invention may, for example, very suitably be carried out by mixing with a $C_5^-$ fraction the reaction product from the first stage, which consists of dimethyl ether, water and unconverted methanol, converting the resultant mixture in the second stage, separating the reaction product from the second stage after cooling into water, a $C_5^-$ fraction and an aromatic hydrocarbon mixture boiling in the gasoline range, and recycling a portion of the $C_5^-$ fraction to the second stage. If the process is carried out in this way the heating up of the methanol feed for the first stage and of the $C_5^-$ fraction envisaged as diluent in the second stage may very suitably be carried out by indirect heat exchange with the hot reaction product from the second stage. To this end the hot reaction product from the second stage is preferably divided into two portions in such a way that one portion is sufficient for the heating up of the methanol feed to the desired reaction temperature and the other portion is sufficient for the heating up of the $C_5^-$ recycle stream.

In the process according to the invention a product is obtained which contains durene. If the durene content of the prepared hydrocarbon mixture amounts to more than a few percent and this mixture is intended to be used as gasoline, the durene content has to be reduced. This may be effected by splitting the aromatic hydrocarbon mixture into a $C_9^-$ fraction and a $C_{10}^+$ fraction, removing durene from the $C_{10}^+$ fraction, for example by crystallization, and mixing the remaining $C_{10}^+$ fraction with the $C_9^-$ fraction. Since the crystalline silicate-containing catalysts which are used in the second stage of the process possess the property to convert durene which is present in the feed above a certain concentration into useful gasoline components, the durene separated from the reaction product of the second stage or the durene-containing $C_{10}^+$ fraction separated from the reaction product of the second stage is preferably recycled to the second stage.

Temperature control of the process according to the invention may also very suitably be carried out by internal or external cooling of the reactor or reactors in which the second stage is performed. External cooling may for example, be carried out by performing the second stage of the process in an apparatus in which a number of tubular reactors in parallel arrangement are present, each reactor containing a fixed catalyst bed, and by surrounding the reactors with a coolant. Internal cooling may for example be carried out by performing the second stage of the process in a reactor in which a number of fixed catalyst beds are present, and by injecting a coolant between the catalyst beds. If the second stage of the process is carried out by passing the feed upwardly through a vertically arranged reactor which contains a fluidized catalyst bed while using catalyst recirculation, internal cooling can be effected by injecting part of the catalyst to be recycled and/or a coolant into the fluidized catalyst bed at different levels. When using internal cooling, the cooling liquid used can very suitably be methanol and/or dimethyl ether separated from the reaction product of the first stage or a durene-containing $C_{10}^+$ fraction separated from the reaction product of the second stage.

The crystalline silicates which are used as catalysts in the second stage of the process according to the invention are as a rule prepared starting from an aqueous mixture containing the following compounds in a given ratio: one or more compounds of an alkali metal, one or more compounds which contain an organic cation or from which such a cation is formed during the preparation of the silicate, one or more silicon compounds, one or more iron compounds and possibly one or more aluminium, gallium and/or germanium compounds. The preparation is effected by maintaining the mixture at elevated temperature until the silicate has been formed and by subsequently separating the silicate crystals from the mother liquor. Before being used in the process according to the invention the organic cations introduced during the preparation should be converted into hydrogen ions by calcination. In the process use is preferably made of silicates having an alkali metal content of less than 1% by weight and in particular less than 0.05% by weight. Such silicates can be prepared from the above-mentioned calcined silicates by ion exchange, for example with an aqueous solution of an ammonium salt, followed by calcination.

Aromatic hydrocarbon mixtures prepared from methanol using a catalyst containing a crystalline silicate according to the invention are very suitable for use as motor gasoline. This applies both to the mixtures which have been prepared according to the two-stage process of the invention and to those prepared according to the single-stage process. It has been found that the quality of these mixtures for use as motor gasoline can be further improved by incorporating natural gas condensate. Motor gasolines composed of 70—85% by volume of the said aromatic mixtures and 15—30% by volume of natural gas condensate are preferred. Preference is further given to such motor gasolines to which n-butane has also been added  well, in particular motor gasolines composed of 60—80% by volume of said aromatic mixtures, 20–  )% by volume of natural gas condensate and up to 10% by volume of n-butane.

The invention will now be further elucidated with reference to the following example.

A crystalline iron silicate (silicate A) was prepared as follows. A mixture of $Fe(NO_3)_3$, $SiO_2$, $NaNO_3$ and $[(C_3H_7)_4N]OH$ in water having the molar composition $Na_2O. 1.5[(C_3H_7)_4N]_2O. 0.125 Fe_2O_3. 25 SiO_2. 468 H_2O$ was heated at 150°C and autogenous pressure for 48 hours in an autoclave. After cooling of the reaction mixture the resultant silicate was filtered off, washed with water until the pH of the wash water was approximately 8 and dried for 2 hours at 120°C. The silicate A thus prepared had the following chemical composition: $0.8[(C_3H_7)_4N]_2O. 0.3 Na_2O. Fe_2O_3. 200 SiO_2. 55 H_2O$. The silicate had an X-ray powder diffraction pattern substantially as mentioned in Table B of the Netherlands patent application No. 7613957. The silicate was thermally stable up to temperatures in excess of 900°C and was capable, after dehydration at 400°C in vacuo, of adsorbing 7% by weight of water at 25°C and saturated water vapour pressure. Starting from silicate A a silicate B was prepared by consecutively calcining silicate A at 500°C, boiling it with 1.0 molar $NH_4NO_3$ solution, washing it with water, reboiling it with 1.0 molar $NH_4NO_3$ solution and by washing it, drying it at 120°C and calcining it at 500°C. In the following Example the dimethyl ether is formed in a first stage by contacting methanol at elevated temperature with a dehydration catalyst.

## Example

Silicate B was used as catalyst in the preparation of an aromatic hydrocarbon mixture from methanol and from dimethyl ether. To this end the relevant feed was passed at a pressure of 5 bar upwardly through a cylindrical, vertically arranged reactor which contained a fixed catalyst bed of silicate B, which had been brought to a temperature of 375°C. In the experiment with methanol as feed, the space velocity was 1 $l.l^{-1}.h^{-1}$. In the experiment with dimethyl ether as feed, a space velocity was used which, expressed in molar terms amounted to half of that used in the experiment with methanol as feed. The results of both experiments are given in the table below.

| Feed | methanol | dimethylether |
|---|---|---|
| Temperature in cat. bed 30 min. after the start of the experiment, °C | | |
| top | 394 | 382 |
| middle | 379 | 374 |
| bottom | 373 | 372 |
| Molecular weight distribution (%w on $C_1^+$) | | |
| $C_1$ | 2.0 | 1.8 |
| $C_2$ | 8.5 | 9.7 |
| $C_3$ | 6.8 | 4.2 |
| $C_4$ | 7.4 | 4.6 |
| $C_5^+$ | 75.3 | 79.7 |
| Total conversion, % | 99 | 99 |
| Conversion to hydrocarbons (% of total conversion) | 97 | 99 |

## Claims

1. A process for the preparation of an aromatic hydrocarbon mixture from methanol, characterized in that the process is carried out in two stages in which dimethyl ether is prepared in the first stage by contacting methanol at elevated temperature with a dehydration catalyst and in which in the second stage an aromatic hydrocarbon mixture is prepared by contacting dimethyl ether from the first stage at elevated temperature with a catalyst which contains a crystalline silicate which:

a) is thermally stable up to temperatures above 600°C,

b) is capable, after dehydration at 400°C in vacuo, of adsorbing more than 3% by weight of water at 25°C and saturated water vapour pressure, and

c) possesses in the dehydrated form the following overall composition, expressed in moles of the oxides, $(1.0 \pm 0.3)(R)_{2/n}O . [a\ Fe_2O_3 . b\ Al_2O_3 . c\ Ga_2O_3] . y\ (d\ SiO_2 . e\ GeO_2)$, wherein

$R$ = one or more mono- or bivalent cations,

$a \geqslant 0.1$,

$b \geqslant 0$,

$c \geqslant 0$,

$a + b + c = 1$,

$y \geqslant 10$,

$d \geqslant 0.1$,

$e \geqslant 0$,

$d + e = 1$, and

$n$ = the valency of R.

2. A process as claimed in claim 1, characterized in that the dehydration catalyst used in gamma alumina.

3. A process as claimed in claim 1 or 2, characterized in that the first stage is carried out at a temperature between 250° and 400°C, a pressure between 1 and 50 bar and a space velocity between 0.63 and 253 $l.l^{-1}.h^{-1}$.

4. A process as claimed in any one of claims 1—3, characterized in that the catalyst which is used in the second stage contains a crystalline silicate of which c and e in the formula giving the overall composition equal 0.

5. A process as claimed in any one of claims 1—4, characterized in that the catalyst which is used

5

in the second stage contains a crystalline silicate of which a in the formula indicating the overall composition is greater than 0.5.

6. A process as claimed in any one of claims 1—5, characterized in that the catalyst which is used in the second stage contains a crystalline silicate of which y in the formula indicating the overall composition is less than 800.

7. A process as claimed in any one of claims 1—6, characterized in that the catalyst which is used in the second stage contains a crystalline silicate having an alkali metal content of less than 1% by weight.

8. A process as claimed in any one of claims 1—7, characterized in that the second stage is carried out at a temperature between 250 and 500°C, a pressure between 0.5 and 100 bar, and a space velocity between 0.1 and 20 $l.l^{-1}.h^{-1}$.

9. A process as claimed in any one of claims 1—8, characterized in that a gaseous light hydrocarbon fraction is added as diluent to the feed for the second stage.

10. A process as claimed in any one of claims 1—9, characterized in that durene or a durene-containing $C_{10}^+$ fraction is separated from the reaction product of the second stage and recycled to the second stage.

## Revendications

1. Un procédé pour la préparation d'un mélange d'hydrocarbures aromatiques à partir de méthanol, caractérisé en ce que le procédé est mis en oeuvre en deux étapes où on prépare de l'oxyde de méthyle dans la première étape en mettant en contact le méthanol à température élevée avec un catalyseur de déshydratation et dans la deuxième étape on prépare un mélange d'hydrocarbures aromatiques en mettant en contact l'oxyde de méthyle provenant de la première étape à température élevée avec un catalyseur qui contient un silicate cristallin qui:
a) est thermiquement stable jusqu'à des températures de plus de 600°C;
b) est capable, après déshydratation à 400°C sous vide, d'adsorber plus de 3% en poids d'eau à 25°C et à la pression de vapeur saturante; et
c) possède dans la forme déshydratée la composition d'ensemble suivante, exprimée en moles des oxydes: $(1,0\pm0.3)(R)_{2/n}O.$ [a $Fe_2O_3$. b $Al_2O_3$. c $Ga_2O_3$].
y (d $SiO_2$. e $GeO_2$), dans laquelle:
R = un ou plusieurs cations mono- ou bivalents,
a ⩾ 0,1,
b ⩾ 0,
c ⩾ 0,
a + b + c = 1,
y ⩾ 10,
d ⩾ 0,1,
e ⩾ 0,
d + e = 1 et
n = la valence de R.

2. Un procédé selon la revendication 1, caractérisé en ce que le catalyseur de déshydratation utilisé est de la gamma-alumine.

3. Un procédé selon l'une des revendications 1 ou 2, caractérisé en ce que la première étape est conduite à une température comprise entre 250 et 400°C, une pression comprise entre 1 et 50 bars et une vitesse spatiale comprise entre 0,63 et 253 $l.l^{-1}.h^{-1}$.

4. Un procédé selon l'une quelconque des revendications 1 à 3, caractérisé en ce que le catalyseur qui est utilisé dans le deuxième étape contient un silicate cristallin pour lequel c et e dans la formule donnant la composition d'ensemble sont chacun zéro.

5. Un procédé selon l'une quelconque des revendications 1 à 4, caractérisé en ce que le catalyseur qui est utilisé dans la deuxième étape contient un silicate cristallin pour lequel a dans la formule indiquant la composition d'ensemble est supérieur à 0,5.

6. Un procédé selon l'une quelconque des revendications 1 à 5, caractérisé en ce que le catalyseur qui est utilisé dans la deuxième étape contient un silicate cristallin pour lequel y dans la formule indiquant la composition d'ensemble est inférieur à 800.

7. Un procédé selon l'une quelconque des revendications 1 à 6, caractérisé en ce que le catalyseur qui est utilisé dans la deuxième étape contient un silicate cristallin ayant une teneur en métal alcalin de moins de 1% en poids.

8. Un procédé selon l'une quelconque des revendications 1 à 7, caractérisé en ce que la deuxième étape est conduite à une température comprise entre 250 et 500°C, une pression comprise entre 0,5 et 100 bars et une vitesse spatiale comprise entre 0,1 et 2,0 $l.l^{-1}.h^{-1}$.

9. Un procédé selon l'une quelconque des revendications 1 à 8, caractérisé en ce qu'une fraction légère d'hydrocarbures gazeuse est ajoutée comme diluant à la charge pour la deuxième étape.

10. Un procédé selon l'une quelconque des revendications 1 à 9, caractérisé en ce qu'on sépare du durène ou une fraction $C_{10}^+$ contenant du durène du produit de réaction de la deuxième étape et on recycle ce durène ou cette fraction à la deuxième étape.

**Patentansprüche**

1. Verfahren zur Herstellung eines aromatischen Kohlenwasserstoffgemisches aus Methanol, dadurch gekennzeichnet, dass das Verfahren in zwei Stufen durchgeführt wird, wobei Dimethyläther in der ersten Stufe durch Kontaktieren von Methanol mit einem Entwässerungskatalysator bei erhöhter Temperatur hergestellt wird und in der zweiten Stufe eine aromatisches Kohlenwasserstoffgemisch durch Kontaktieren des Dimethyläthers aus der ersten Stufe bei erhöhter Temperatur mit einem Katalysator hergestellt wird, der ein kristallines Silikat enthält, das

a) bei Temperaturen über 600°C thermisch stabil ist,

b) nach dem Wasserentzug bei 400°C unter vermindertem Druck über 3 Gewichtsprozent Wasser bei 25°C und gesättigtem Wasserdampfdruck adsorbieren kann und

c) in entwässerter Form folgende Zusammensetzung hat, angegeben in Mol der Oxide:

$(1.0 \pm 0{,}3)(R)_{2/n}O$. [a $Fe_2O_3$. b $Al_2O_3$. c $Ga_2O_3$].

y (d $SiO_2$. e $GeO_2$), wobei

R ein oder mehrere ein- oder zweizertige(s) Kation(en)

$a \geqslant 0{,}1$,

$b \geqslant 0$,

$c \geqslant 0$,

$a + b + c = 1$,

$y \geqslant 10$,

$d \geqslant 0{,}1$,

$e \geqslant 0$,

$d + e = 1$ und

n die Wertigkeit von R ist.

2. Verfahren gemäss Anspruch 1, dadurch gekennzeichnet, dass der verwendete Entwässerungskatalysator $\gamma$-Aluminiumoxid ist.

3. Verfahren gemäss Anspruch 1 oder 2, dadurch gekennzeichnet, dass die erste Stufe bei einer Temperatur zwischen 250 und 400°C, einem Druck zwischen 1 und 50 bar und einer Raumgeschwindigkeit zwischen 0,63 und 253 $l.l^{-1}.h^{-1}$ durchgeführt wird.

4. Verfahren gemäss einem der Ansprüche 1 bis 3, dadurch gekennzeichnet, dass der in der zweiten Stufe verwendete Katalysator ein kristallines Silikat enthält, in dem c und e in der die Gesamtzusammensetzung angebenden Formel O bedeuten.

5. Verfahren gemäss einem der Ansprüche 1 bis 4, dadurch gekennzeichnet, dass der in der zweiten Stufe verwendete Katalysator ein kristallines Silikat enthält, in dem a in der die Gesamtzusammensetzung angebenden Formel grösser als 0,5 ist.

6. Verfahren gemäss einem der Ansprüche 1 bis 5, dadurch gekennzeichnet, dass der in der zweiten Stufe verwendete Katalysator ein kristallines Silikat enthält, in dem y in der die Gesamtzusammensetzung angebenden Formel kleiner als 800 ist.

7. Verfahren gemäss einem der Ansprüche 1 bis 6, dadurch gekennzeichnet, dass der in der zweiten Stufe verwendete Katalysator ein kristallines Silikat mit einem Alkalimetallgehalt unter 1 Gewichtsprozent enthält.

8. Verfahren gemäss einem der Ansprüche 1 bis 7, dadurch gekennzeichnet, dass die zweite Stufe bei einer Temperatur zwischen 250 und 500°C, einem Druck zwischen 0,5 und 100 bar und einer Raumgeschwindigkeit zwischen 0,1 und 20 $l.l^{-1}.h^{-1}$ durchgeführt wird.

9. Verfahren gemäss einem der Ansprüche 1 bis 8, dadurch gekennzeichnet, dass eine gasförmige leichte Kohlenwasserstoffraktion als Verdünningsmittel der Zuspeisung für die zweite Stufe zugesetzt wird.

10. Verfahren gemäss einem der Ansprüche 1 bis 9, dadurch gekennzeichnet, dass Durol oder eine Durol enthaltende $C_{10}^+$-Fraktion vom Reaktionsprodukt der zweiten Stufe abgetrennt und in die zweite Stufe zurückgeführt wird.